# EUROPEAN PATENT SPECIFICATION

(11) **EP 4 017 990 B1**
(45) Date of publication and mention of the grant of the patent: **08.10.2025**
(21) Application number: 20854668.9
(22) Date of filing: 20.08.2020
(51) Int. Cl.: C12Q 1/04, C12Q 1/18, G01N 33/58, C12N 13/00, H01J 49/16, C12Q 1/02

(54) **METHODS FOR SCREENING AND SUBSEQUENT PROCESSING OF SAMPLES TAKEN FROM NON-STERILE SITES**
VERFAHREN ZUM SCREENEN UND NACHFOLGENDEN VERARBEITEN VON PROBEN AUS UNSTERILEN STELLEN
PROCÉDÉS DE CRIBLAGE ET DE TRAITEMENT ULTÉRIEUR D'ÉCHANTILLONS PRÉLEVÉS SUR DES SITES NON STÉRILES

(30) Priority: 20.08.2019 US 201962889414 P
(43) Date of publication of application: 29.06.2022
(73) Proprietor: Pattern Bioscience, Inc., Austin, Texas 78759 (US)
(72) Inventor: ARAB, Nicolas, Austin, Texas 78759 (US); JOHNSON, Ross, Austin, Texas 78759 (US)
(74) Representative: Eisenführ Speiser
(86) International application number: PCT/US2020/047110
(87) International publication number: WO 2021/035009

(56) References cited:
- US-A1- 2016 237 469
- US-A1- 2017 309 462
- US-A1- 2018 172 675
- US-A1- 2018 203 005
- MAUGERI GAETANO ET AL: "Identification and Antibiotic-Susceptibility Profiling of Infectious Bacterial Agents: A Review of Current and Future Trends", vol. 14, no. 1, 1 January 2019 (2019-01-01), DE, pages 1700750, XP093036917, ISSN: 1860-6768, Retrieved from the Internet <URL:https://www.ncbi.nlm.nih.gov/pmc/articles/PMC6330097/pdf/emss-79853.pdf> DOI: 10.1002/biot.201700750
- SHIN DONG JIN ET AL: "Emerging Analytical Techniques for Rapid Pathogen Identification and Susceptibility Testing", 12 June 2019 (2019-06-12), XP055788241, Retrieved from the Internet <URL::10.1146/annurevanchem-061318-115> [retrieved on 20210322], DOI: 10.1146/annurev
- LI YIYAN ET AL: "Emerging Microtechnologies and Automated Systems for Rapid Bacterial Identification and Antibiotic Susceptibility Testing", SOCIETY FOR LABORATORY AUTOMATION AND SCREENING, vol. 22, no. 6, 29 August 2017 (2017-08-29), pages 585 - 608, XP055612422, DOI: 10.1177/2472630317727519
- KAUSHIK ANIRUDDHA M ET AL: "Accelerating bacterial growth detection and antimicrobial susceptibility assessment in integrated picoliter droplet platform", BIOSENSORS AND BIOELECTRONICS, ELSEVIER SCIENCE LTD, UK, AMSTERDAM , NL, vol. 97, 27 June 2017 (2017-06-27), pages 260 - 266, XP085112957, ISSN: 0956-5663, DOI: 10.1016/J.BIOS.2017.06.006

## Description

### FIELD OF THE INVENTION

The present invention relates generally to the identification and phenotypic analysis of microorganisms and, more particularly but without limitation, to identifying pathogenic microorganisms in samples taken from non-sterile sites and determining a phenotypic response thereof to one or more test reagents using droplet microfluidics.

### BACKGROUND OF THE INVENTION

Analysis of samples taken from non-sterile sites can pose challenges because those samples may include both pathogenic and commensal microorganisms. For example, to determine appropriate patient care, the identity of the pathogenic microorganisms may need to be identified and the response of the pathogen-rather than the response of the commensal microorganisms-to various drugs (e.g., antibiotics) may need to be assessed. Current techniques to identify pathogens in non-sterile samples, such as quantitative culture, quantitative polymerase chain reaction (QPCR), and nucleic acid amplification tests (NAATs), may be inefficient, expensive, and complex. For example, in quantitative culture, it can take about 1 to 2 days to culture and allow the microorganisms in the sample to achieve identifiable growth. QPCR is typically expensive and may only be able to identify some pathogens. And quantitative culture, QPCR, and NAATs generally must be performed by skilled personnel due to the complicated work flow associated with each of the techniques.

Conventional processes used to, for example, determine the susceptibility of a pathogen to antibiotics (e.g., antibiotic susceptibility tests (ASTs)) can be inefficient, timeintensive, and/or inaccurate when commensal microorganisms are also present in the sample. Phenotypic test methods such as broth microdilution and disk diffusion generally require additional culturing of the sample, which can lengthen the amount of time required for analysis. Additionally, in these processes, the pathogenic microorganisms must be isolated from commensal microorganisms (e.g., by streaking the sample across a plate), which can be time- and work-intensive and require skilled personnel. Genotypic test methods, such as NAATs, may be less accurate than phenotypic methods because they assess the response of the pathogen indirectly based on genetic information and may not be able to analyze all species of pathogens or account for genetic mutations. For example, NAATs generally target molecular markers indicative of resistance mechanisms. To do so, a unique primer may have to be prepared for each of the relevant markers. When there are a large amount of markers, developing specific primers for each can be challenging and, without a suitable primer, a relevant marker may be missed. And resistance mechanisms can evolve, something NAATs may not be able to take into account. US 2018/0203005 Al relates to a microfluidic droplet-based bioassay platform that offers single cell based analysis for monitoring of bacterial infection and cell function, as well as for high throughput testing of the susceptibility of single cells to antibiotics and other pharmaceutical agents.

US 2018/0172675 A1 relates to compositions and methods for rapid and sensitive identification of disease-causing cells and the rapid and sensitive characterization of their response to the drugs used to treat them.

### SUMMARY OF THE INVENTION

There accordingly is a need in the art for methods of analyzing a sample taken from a non-sterile site in a rapid, cost-effective, and efficient manner. The present methods as set out in the appended claims can address this need through the use of droplet microfluidics. Further aspects are disclosed as follows. These aspects only constitute the claimed invention if they fall under the scope of the appended claims and are otherwise to assist understanding of the invention. A first portion of a sample can be analyzed to identify and quantify the microorganism(s) therein at least by generating a plurality of first droplets from a first liquid that includes a viability indicator and the first portion of the sample. Each of one or more microorganisms of the first portion of the sample can be encapsulated within one of the first droplets, which can have a relatively low volume (e.g., on the order of nanoliters or picoliters) such that the concentration of the encapsulated microorganism(s) can be relatively high. This may allow the first portion of the sample to be analyzed without the lengthy culture that is performed in quantitative culture and QPCR. And droplet generation can be performed with a first microfluidic chip that is simple to load.

To identify and quantify the microorganism(s), each of the encapsulating first droplets includes a viability indicator and a single species such that the droplet has a characteristic signature (e.g., a fluorescence that changes over time) that is, at least in part, attributable to the encapsulated species. In this manner, droplets that encapsulate different species can have different signatures, permitting differentiation thereof. A first set of data that includes these characteristic signature(s) can be captured and analyzed to ascertain the identity (e.g., based on the characteristic signature(s)) and quantity (e.g., based on the number of droplets exhibiting a particular microorganism-induced signature) of microorganism(s) of the first portion of the sample. At least one of the species can be identified as a target (e.g., pathogenic) species based on this data (e.g., if the data indicates the concentration of the species in the sample is above a threshold concentration)

If the test is negative (e.g., no pathogens are detected), further analysis need not be performed to save the expense of further tests. If a target species is identified, a second portion of the sample can be analyzed to ascertain a phenotypic response of the target species to one or more test reagents, such as the target species' susceptibility to one or more antibiotics. This analysis can be performed using droplet microfluidics in substantially the same manner as described above. For each of one or more aliquots of the second portion of the sample, a plurality of second droplets can be generated from a second liquid that includes the aliquot such that each of one or more microorganisms of the aliquot is encapsulated within one of the second droplets. Each of the second droplets includes a viability indicator (e.g., the same used for the above-described identification and quantification) and a test reagent can be introduced into at least some of the second droplets. A second set of data that includes the resulting characteristic signature(s) of the encapsulating second droplets can be captured. The test reagent may affect the characteristic signature of each of the encapsulating droplets (e.g., by killing or inhibiting the growth of microorganism(s) disposed therein)-the phenotypic response of the encapsulated microorganism(s) can be determined based on whether these variations are present.

To determine the phenotypic response of the target species, the second set of data can be referenced to the first set of data. Because the phenotypic analysis may be performed after the initial screen, the relative concentrations of the microorganism(s) in the second portion of the sample may be different from those in the original sample (e.g., because the microorganism(s) can replicate). For example, when multiple species of microorganisms are present in the sample, the species can have different replication rates-a commensal microorganism having a relatively fast replication rate may appear pathogenic in the second portion of the sample. By referencing the second set of data to the first set of data (which can provide a better indication of the original microorganism concentrations), the relevant species for investigation-and thus the relevant characteristic signature-can be identified such that the phenotypic test can appropriately assess the effect of the test reagent on the target (e.g., pathogenic), rather than non-target (e.g., commensal), species. Because this approach permits differentiation between droplets that encapsulate different species, time- and work-intensive isolation (e.g., by streaking) need not be performed, making the test more efficient than quantitative culture and QPCR. And because the analysis is phenotypic, it can be more accurate than NAATs.

Mass spectrometry can also be used to identify microorganism(s) with higher resolution after the initial screen. For example, at least some of the first droplets can be removed from the microfluidic chip and disposed on a plate. The location of one(s) of the removed first droplets that encapsulate microorganism(s) can be ascertained to determine where to begin scanning and thereby accelerate the analysis. The droplets can be dried and the encapsulated microorganism(s) can be lysed in preparation for mass spectrometry. The mass spectrometer can be a matrix assisted laser desorption/ionization time of flight (MALDI-TOF) mass spectrometer.

Preferred aspects of the claimed invention are disclosed in the dependent claims.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. Views in the figures are drawn to scale, unless otherwise noted, meaning the sizes of the depicted elements are accurate relative to each other for at least the embodiment in the view.
**FIG. 1** illustrates some of the present methods of screening and analyzing a sample using droplet microfluidics and, optionally, mass spectrometry.
**FIG. 2** is a schematic of a system that can be used to perform at least some of the methods of FIG. 1.
**FIGs. 3A** and **3B** are schematics of a chip defining a microfluidic network configured to generate droplets from a first portion of a sample. The chip is shown in use, with a liquid that includes the first portion of the sample being disposed in an inlet port of the chip (FIG. 3A) and directed to a test volume of the microfluidic network such that droplets are generated (FIG. 3B). The droplets can be analyzed in the test volume to determine the identity and quantity of microorganism(s) of the first portion of the sample.
**FIG. 3C** is a graph showing measurements that can be obtained when droplets generated from the first portion of the sample are analyzed. The illustrated measurements include the fluorescence of encapsulating droplets over time (relative to that of nonencapsulating droplets), which can be used to identify the species of encapsulated microorganism(s) and the quantity thereof.
**FIG. 4A** is an exploded perspective exploded view of an embodiment of a first microfluidic chip that can be used for the analysis described in reference to FIGs. 3A and 3B.
**FIG. 4B** is a top view of the chip of FIG. 4A showing the inlet ports thereof.
**FIG. 4C** is a bottom view of a first piece of the chip of FIG. 4A, with a second piece of the chip removed. FIG. 4C illustrates the microfluidic networks defined by the chip.
**FIG. 4D** is an enlarged view of one of the microfluidic networks of the chip of FIG. 4A.
**FIG. 4E** is a sectional view of the chip of FIG. 4A taken along line 4E-4E of FIG. 4B. FIG. 4E illustrates the inlet port of one of the chip's microfluidic networks and a portion of a flow path connected thereto.
**FIG. 4F** is an enlarged view of one of the droplet-generating region(s) of one of the microfluidic networks of the chip of FIG. 4A. In the droplet-generating region, a flow path includes a constricting section, a constant section, and an expanding section such that a minimum cross-sectional area of the flow path increases along the flow path.
**FIG. 4G** is a partial sectional view of the chip of FIG. 4A taken along line 4G-4G of FIG. 4F. FIG. 4G illustrates the relative sizes of the constricting section and an upstream channel connected to the constricting section.
**FIG. 4H** is a partial sectional view of the microfluidic chip of FIG. 4A taken along line 4H-4H of FIG. 4F. FIG. 4H illustrates the geometry of the constant and expanding sections relative to the constricting section, the expanding section having a ramp defined by a single planar surface.
**FIG. 5** is a partial sectional view of a droplet-generating region of another embodiment of the present microfluidic chips that is substantially similar to the chip of FIG. 4A, the primary exception being that the ramp of the expanding section in the FIG. 5 chip is defined by a plurality of steps.
**FIGs. 6A-6D** are schematics illustrating droplet generation in the chip of FIG. 4A when liquid flows from the constricting section into the constant and expanding sections.

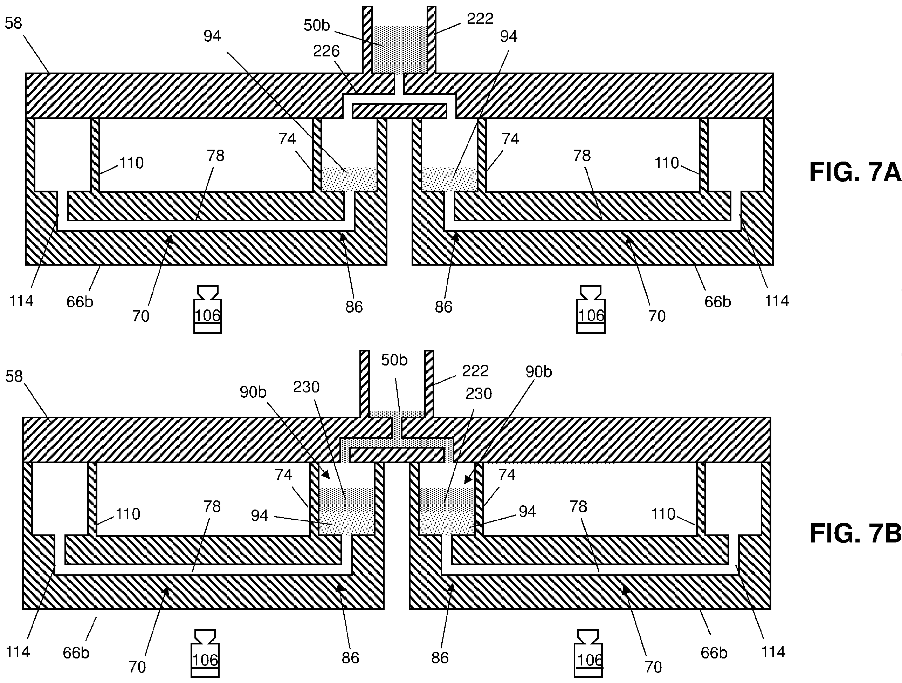

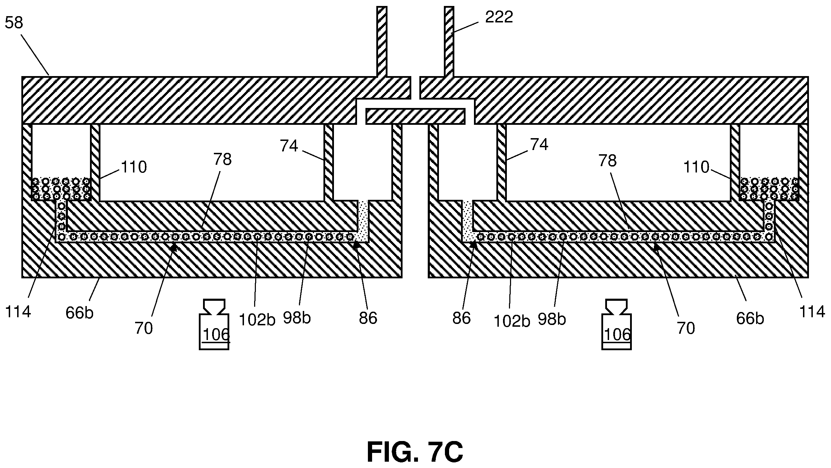

**FIGs. 7A-7C** are schematics of a second device, in use, that is configured to partition a second portion of the sample into one or more aliquots (FIGs. 7A and 7B) and, for each of the aliquot(s), generate droplets from a liquid including the aliquot (FIG. 7C). The second device can include one or more microfluidic chips that are substantially the same as those used to generate droplets from the liquid including the first portion of the sample such that droplets from the aliquot-containing liquid can be generated in substantially the same manner. The second device can be configured such that a test reagent can be introduced into the droplets, which can be analyzed in a test volume to determine a phenotypic response thereof to the test reagent.
**FIG. 8A** is a perspective view of an embodiment of the second device that can be used for the analysis described in reference to FIGs. 7A-7C.
**FIG. 8B** is a bottom view of the second device of FIG. 8A showing the microfluidic chips thereof, each of which can be substantially similar to the microfluidic chip of FIG. 4A.
**FIG. 8C** is a top view of the second device of FIG. 8A, which shows an injection port of the second device that can receive the second portion of the sample.
**FIG. 8D** is a side view of the second device of FIG. 8A.
**FIG. 8E** is a sectional view of the second device of FIG. 8A taken along line 8E-8E of FIG. 8C. FIG. 8E illustrates the injection port of the device and a channel connected thereto through which the second portion of the sample can flow towards the microfluidic chips. The second device can include piercers, each configured to break a seal of a respective one of the inlet ports of the chips such that an aliquot can be introduced therein.
**FIG. 8F** is a bottom view of the second device of FIG. 8A where a second piece of each of the chips is removed. FIG. 8F illustrates the microfluidic networks of the chips.
**FIG. 8G** is a top view of a bottom piece of the second device of FIG. 8A illustrating channels defined by the second device through which the second portion of the sample can be partitioned into aliquots that can be directed to the microfluidic networks of the chips.
**FIG. 8H** is a schematic showing the arrangement of channels of the second device of FIG. 8A relative to the chips of the second device.
**FIGs. 9A** and **9B** are schematic top and side views, respectively, of a plate with some of the droplets generated using the chip of FIG. 3A disposed thereon. The plate can be used for mass spectrometry.
**FIG. 9C** is a schematic illustrating imaging of the plate of FIG. 9A with the droplets disposed thereon such that the location of microorganism-encapsulating droplets can be determined.
**FIG. 9D** is a schematic illustrating microorganism(s) remaining on the plate of FIG. 9A after the droplets are dried.
**FIG. 9E** is a schematic illustrating application of a lysing reagent onto the plate of FIG. 9A to lyse the microorganism(s) disposed thereon.
**FIG. 9F** is a schematic illustrating a matrix material disposed on the plate of FIG. 9A and mixed with the microorganism(s).
**FIG. 9G** is a schematic of a MALDI-TOF mass spectrometer in use to analyze the microorganism(s) on the plate of FIG. 9A.

### DETAILED DESCRIPTION OF THE INVENTION

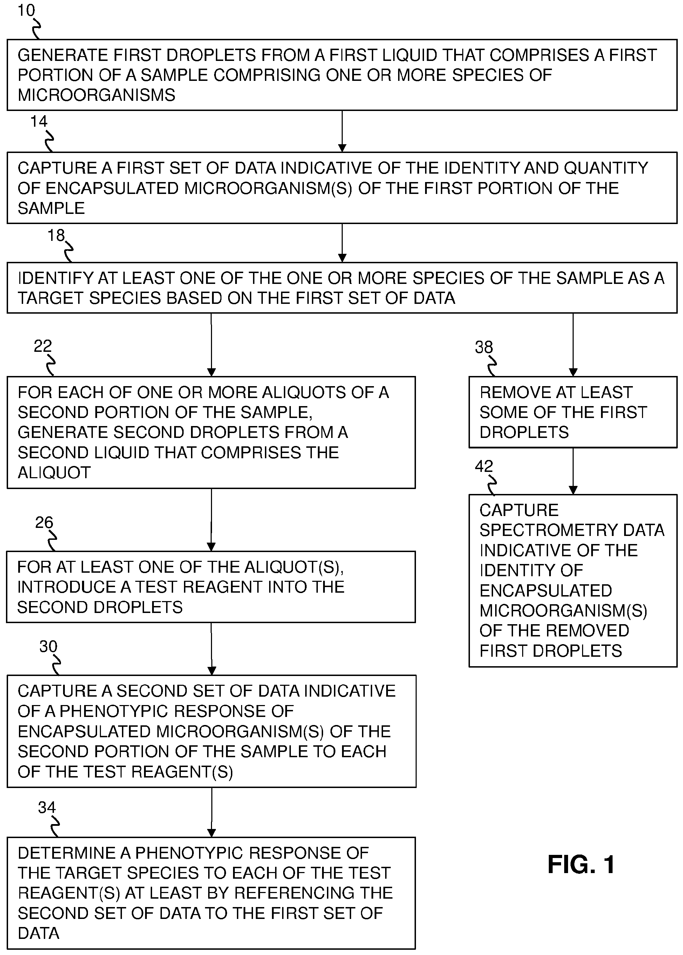

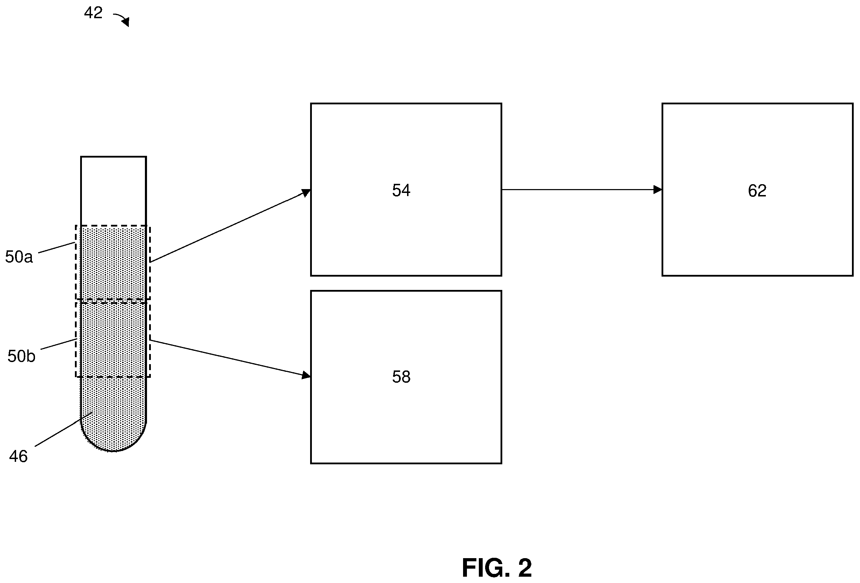

FIG. 1 illustrates some of the present methods of analyzing a sample (e.g., 46) and FIG. 2 is a schematic of a system 42 that can be used to perform some of those methods. While some of the present methods are described with reference to system 42 and illustrative devices thereof (e.g., 54, 58, and 62), system 10 and those devices are not limiting on the present methods, which can be performed using any suitable system.

The sample can comprise one or more-optionally two or more-species of microorganisms, such as one or more species of bacteria and/or fungi, and can be taken from a non-sterile site of a patient. For example, the sample can include urine, sputum, skin, soft tissue, material collected from bronchoalveolar lavage (BAL), material collected from endotracheal aspiration (ETA), and/or the like, and can be an aqueous liquid. Because the sample may be taken from a non-sterile site, it may include both pathogenic and commensal microorganisms. As described in further detail below, sample analysis can be performed to determine whether the sample includes pathogenic microorganisms and, if present, to assess a phenotypic response of the pathogenic microorganisms to one or more test reagents (e.g., antibiotic susceptibility)-as distinguished from that of any commensal microorganisms in the sample-in a cost-effective, fast, and accurate manner, compared to conventional screening and testing techniques. The analysis can include screening a first portion (e.g., 50a) of the sample with a first device (e.g., 54) and (e.g., if pathogenic microorganisms are detected in the screen) testing a second portion (e.g., 50b) of the sample with a second device (e.g., 58) to determine a phenotypic response of the microorganism(s). In some methods the first portion of the sample can be further analyzed with a mass spectrometer (e.g., 62), whether or not phenotypic testing is performed.

The sample can be processed in preparation for the analysis, such as via size filtration. For example, the sample can be filtered using a filter having a pore size that is less than or equal to any one of, or between any two of, 15, 14, 13, 12, 11, 10, 9, 8, 7, or 6 µm (e.g., less than or equal to 10 µm). The sample can also (e.g., instead of size filtration) be centrifuged. To promote microorganism growth, the sample can be suspended in and/or diluted with a broth (e.g., such that the below-described first and/or second liquids comprise a broth).

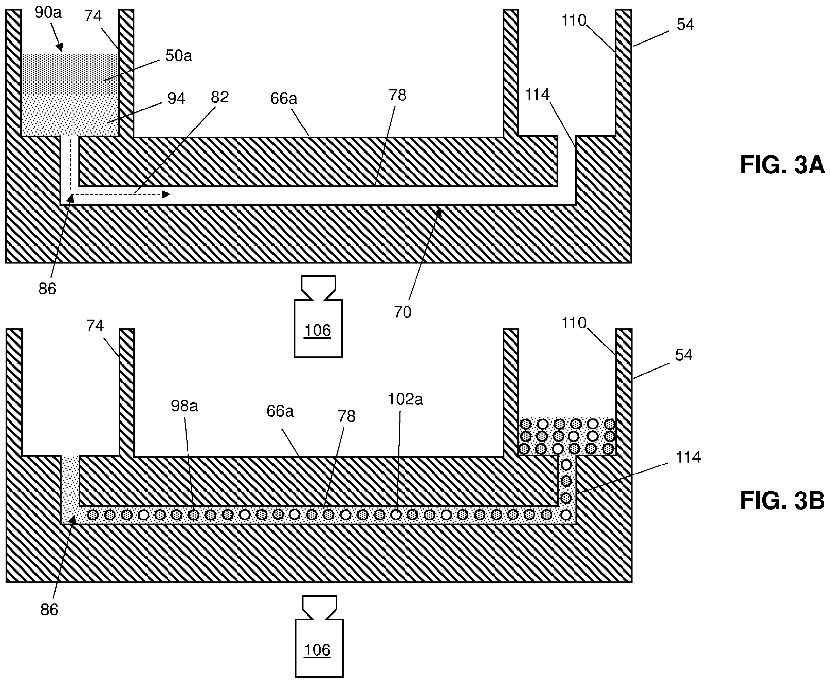

Referring to FIGs. 3A and 3B, to perform the screen, some methods include a step 10 of generating a plurality of first droplets (e.g., 98a) from a first liquid (e.g., 90a) that comprises the first portion of the sample (which can be an aqueous liquid). The first droplets can be generated in any suitable manner, such as with a first chip (e.g., 66a) of the first device, the first chip defining a microfluidic network (e.g., 70) that includes one or more inlet ports (e.g., 74), a test volume (e.g., 78), and one or more flow paths (e.g., 82) extending between the inlet port(s) and the test volume. To generate the first droplets, the first liquid can be disposed within at least one of the inlet port(s) (FIG. 3A) and directed along the flow path(s), through at least one droplet-generating region (e.g., 86), and to the test volume (FIG. 3B). The first liquid can include a non-aqueous liquid (e.g., 94) (e.g., an oil, such as a fluorinated oil, that can include a surfactant) that, in conjunction with the configuration of the droplet-generating region(s), can facilitate droplet generation (e.g., via Laplace pressure gradients), as described in further detail below. To promote droplet generation, the non-aqueous liquid can be relatively dense compared to water, e.g., a specific gravity of the non-aqueous liquid can be greater than or equal to any one of, or between any two of, 1.2, 1.3, 1.4, 1.5, 1.6, or 1.7 (e.g., greater than or equal to 1.5). The microfluidic network can also include one or more outlet ports and one or more outlet channels in fluid communication between the test volume and the outlet port(s) such that at least some of the first droplets flow from the test volume through the outlet channel(s), and into the outlet port(s). These droplets can be used for mass spectrometry, described in further detail below.

As a result of the droplet generation, each of one or more microorganisms of the first portion of the sample can be encapsulated within one of the first droplets. Substantially all of the encapsulating first droplets (e.g., 102) can include a single microorganism (and, optionally, progeny thereof). To facilitate analysis of the microorganism(s), each of the first droplets can have a relatively low volume-such as, for example, less than or equal to any one of, or between any two of, 10,000, 5,000, 1,000, 500, 400, 300, 200, 100, 75, or 25 picoliters (pL) (e.g., between 25 and 500 pL)-such that the concentration of microorganism(s) encapsulated by a first droplet is relatively high regardless of the microorganism concentration in the sample.

Some methods include a step 14 of capturing, with one or more sensors (e.g., 106), a first set of data indicative of the identity and quantity of the encapsulated microorganism(s) of the first portion of the sample (e.g., by analyzing the first droplets that are disposed in the test volume). The first liquid can include a reporter, such as viability indicator, having one or more characteristics (e.g., fluorescence) that change based on droplet conditions that can be affected by microorganism(s) encapsulated therein. Each of the species of microorganisms may affect droplet conditions differently (e.g., due to unique metabolic characteristics of the species) and, as such, each of the encapsulating droplets may exhibit a characteristic signature over time that depends on the species disposed therein. The sensor(s) can detect and measure these signatures, which can be used to assess the identity (e.g., based on the characteristic signature(s)) and quantity (e.g., based on the number of droplets exhibiting a microorganism-induced signature) of microorganism(s) of the first portion of the sample. The relatively low volume of the droplets can facilitate these measurements.

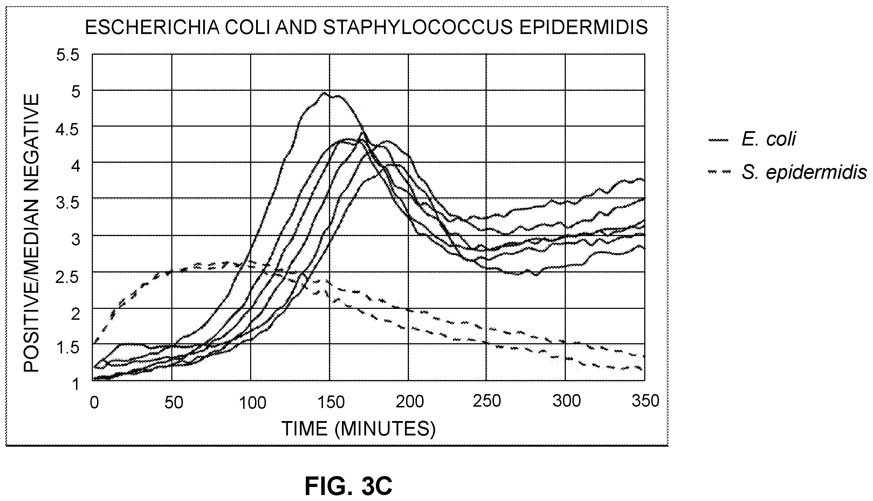

To illustrate, and referring additionally to FIG. 3C, the viability indicator can have a fluorescence that changes based on droplet conditions and the first set of data can comprise measurements of the fluorescence of at least some of the first droplets over a first test period. The viability indicator can comprise, for example, resazurin. Resazurin can have a low fluorescence; however, an encapsulated microorganism-and the progeny thereof-can irreversibly reduce resazurin into resorufin, which may have a fluorescence higher than that of resazurin. Resorufin may in turn be reversibly reduced to non-fluorescent hydroresorufin depending on the reduction potential of the droplet, which may be dictated at least in part on the species of the encapsulated microorganism(s). Each of the encapsulating droplets may accordingly exhibit a characteristic fluorescent signature that varies over time based on the species of microorganism(s) encapsulated therein. The sensor(s), which can comprise imaging sensor(s), can measure this change in fluorescence for each of the encapsulating droplets (e.g., relative to the fluorescence of droplets that do not encapsulate microorganisms), and the number of droplets exhibiting each fluorescent signature can be counted to assess the quantity of each species of microorganism(s) of the first portion of the sample. As shown in FIG. 3C, for example, six droplets encapsulating *E. coli* have a fluorescent signature distinct from that of two droplets encapsulating *S*. *epidermidis.* For each of the species of microorganism(s) in the sample, the identity thereof can be determined at least by referencing the measured characteristic fluorescent signature(s) to a database of known signatures.

While resazurin is one example of a viability indicator that can be used in the screen, in other embodiments the viability indicator can comprise any suitable composition by which each of the encapsulating droplets can exhibit a characteristic signature (e.g., a characteristic fluorescent signature) indicative of the identity of the microorganism(s) encapsulated therein. Suitable viability indicators can comprise, for example, tetrazolium, coumarin, anthraquinone, cyanine, azo, xanthene, arylmethine, a pyrene derivative, a ruthenium bipyridyl complex, and/or the like.

Some methods include a step 18 of identifying at least one of the one or more species of the sample as a target species based on the first set of data. For example, the concentration of each of the one or more species in the sample can be calculated based on the first set of data and, if the concentration is greater than or equal to a threshold concentration-which can, but need not, be different for each of the species-the species can be identified as a target (e.g., pathogenic) species. For each of the species, the concentration can be assessed by determining the proportion of analyzed first droplets (e.g., those in the test volume) that encapsulate microorganisms of that species (e.g., as described above). Species present in concentrations below their respective threshold concentrations may be identified as commensal.

If it is determined that none of the species of microorganisms in the sample is pathogenic (e.g., the concentration thereof is below a threshold concentration), the sample need not be analyzed further (e.g., with the second device or mass spectrometer). By performing the screen in a device separate from that used for phenotypic analysis, sample analysis can be performed cost effectively. Consumables configured for phenotypic analysis (e.g., ASTs) can be relatively expensive, compared to the first chip. These costs may be unnecessary if the sample does not include pathogens-using the inexpensive first chip to make that determination may allow such unnecessary costs to be avoided. As described in further detail below, this multi-device analysis can be performed efficiently at least in part due to the use of the above-described microfluidic droplet analysis.

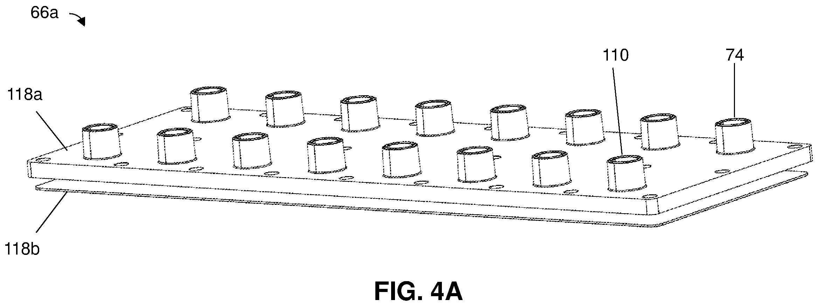

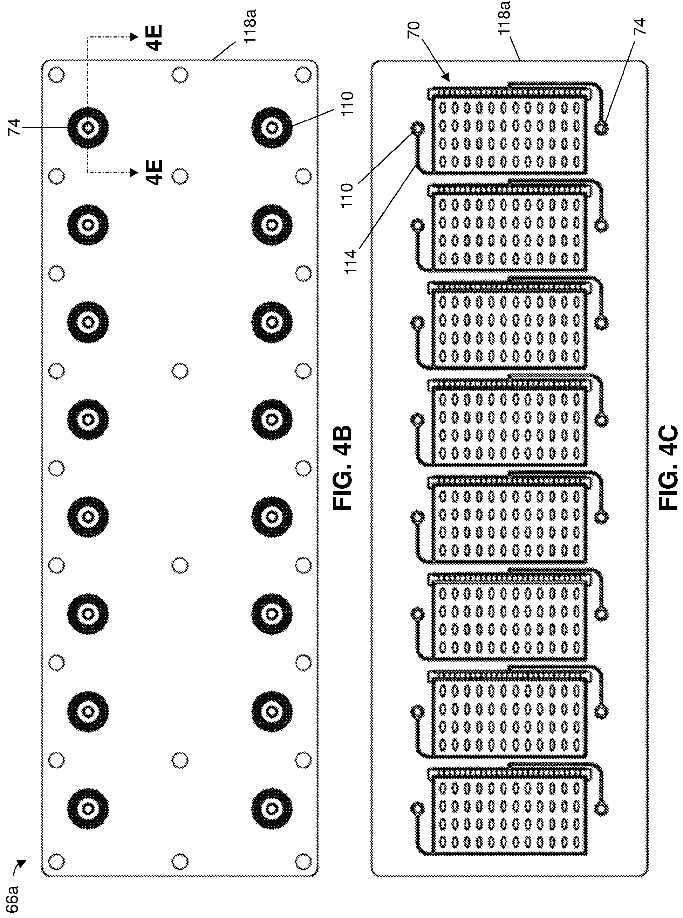

Referring to FIGs. 4A-4H, shown is an illustrative first chip that can be used for the identification and quantification of microorganism(s) of the sample. As shown, the chip defines a plurality of microfluidic networks (e.g., each having inlet port(s), flow path(s), a test volume, outlet channel(s), and outlet port(s) as described above) (FIGs. 4A-4C); in other embodiments, however, the chip can define a single microfluidic network. A multi-network chip may permit simultaneous analysis of multiple samples-for example, as shown, the first chip has eight microfluidic networks and, as such, can be used to analyze eight separate samples. The chip can comprise a single piece or multiples pieces (e.g., first and second pieces 118a and 118b), where at least one of the pieces defines at least a portion of the microfluidic networks. The pieces of the chip can comprise any suitable material; for example, at least one of the first and second pieces can comprise a (e.g., rigid) polymer and, optionally, one of the pieces can comprise a polymeric (e.g., transparent) film.

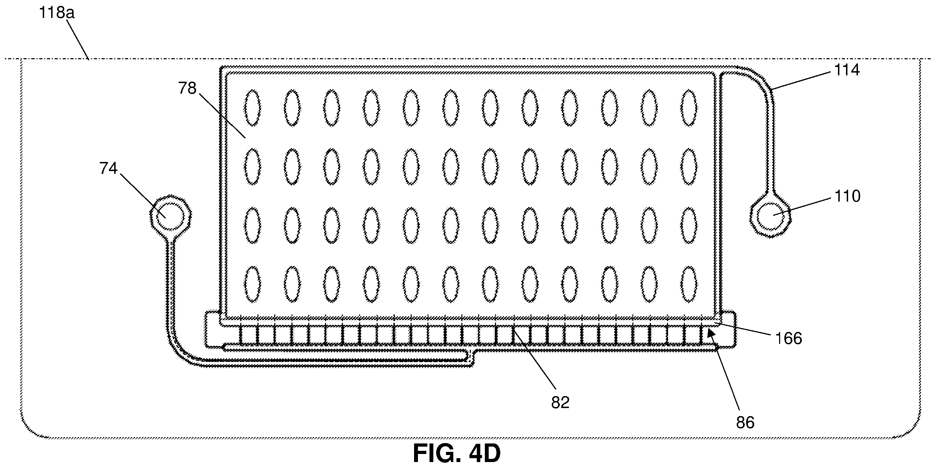

Referring particularly to FIG. 4D, which shows one of the microfluidic networks of the first chip, the flow path(s) can be defined by one or more channels and/or other passageways through which fluid can flow. Each of the flow path(s) can have any suitable maximum transverse dimension to facilitate microfluidic flow, such as, for example, a maximum transverse dimension, taken perpendicularly to the centerline of the flow path, that is less than or equal to any one of, or between any two of, 2,000, 1,500, 1,000, 500, 300, 200, 100, 50, or 25 µm.

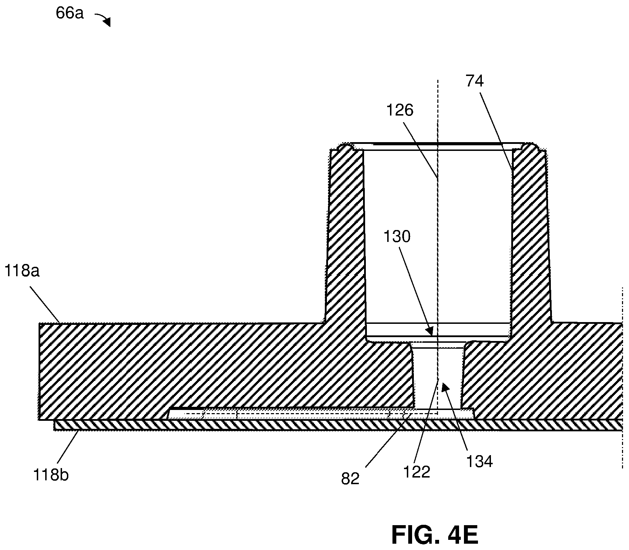

The chip can be configured to permit vacuum loading of the first liquid. For example, before the first liquid is directed to the test volume of one of the microfluidic networks, gas in the test volume can be evacuated at least by reducing pressure at a first one of the inlet port(s) such that the gas flows from the test volume, through at least one of the flow path(s), and out of the first inlet port. The first liquid can be disposed in the first port such that the gas can pass through the liquid. Referring to FIG. 4E, the relative dimensions of the first port and the portion of the flow path connected thereto can facilitate bubble formation as the gas passes through the liquid and can minimize or prevent liquid losses (e.g., that may result if slug flow is produced). For example, that portion of the flow path can have a minimum cross-sectional area (e.g., 134) (taken perpendicularly to centerline (e.g., 122) of the portion) that is smaller than a minimum cross-sectional area (e.g., 130) of the inlet port (taken perpendicularly to centerline (e.g., 26) of the inlet port), e.g., a minimum cross-sectional area that is less than or equal to any one of, or between any two of, 90%, 80%, 66%, 60%, 46%, 40%, 30%, 20%, or 10% (e.g., less than or equal to 90% or 10%) of the minimum cross-sectional area of the inlet port. The smaller cross-sectional area of the portion of the flow path connected to the first inlet port can facilitate formation of gas bubbles having a diameter smaller than that of the inlet port such that slug flow and thus liquid losses are mitigated during gas evacuation. The bubbles can agitate and thereby mix the first liquid to facilitate loading and/or analysis thereof in the test volume.

Prior to the pressure reduction, the pressure at the first port (and, optionally, in the test volume) can be substantially ambient pressure; to evacuate gas from the test volume, the pressure at the first port can be reduced below ambient pressure. For example, reducing pressure can be performed such that the pressure at the first port is less than or equal to any one of, or between any two of, 0.5, 0.4, 0.3, 0.2, 0.1, or 0 atm. Greater pressure reductions can increase the amount of gas evacuated from the test volume. During gas evacuation, the outlet port(s) of the microfluidic network can be plugged (e.g., to prevent the inflow of gas therethrough); in other embodiments, however, the chip can have no outlet ports.

To load the first liquid, pressure at the first port can be increased, optionally such that pressure at the first port is substantially ambient pressure after loading is complete. As a result, the first liquid can flow along the flow path(s) such that, for each of the flow path(s), at least a portion of the first liquid flows from the first port, through at least one droplet-generating region, and into the test volume. As the liquid is introduced into the test volume, the pressure within the test volume can increase until it reaches substantially ambient pressure as well. By achieving pressure equalization between the test volume and the environment outside of the chip (e.g., to ambient pressure), the position of the droplets within the test volume can be maintained for analysis without the need for additional seals or other retention mechanisms. Additionally, a negative pressure gradient can result because the pressure in the test volume can be below that outside of the chip after gas evacuation-this negative pressure gradient can reinforce seals (e.g., between different pieces of the chip) to prevent chip delamination and can contain unintentional leaks by drawing gas into a leak if there is a failure. Leak containment can promote safety when, for example, the first portion of the sample includes pathogens. In other embodiments, however, the chip can be loaded without gas evacuation.

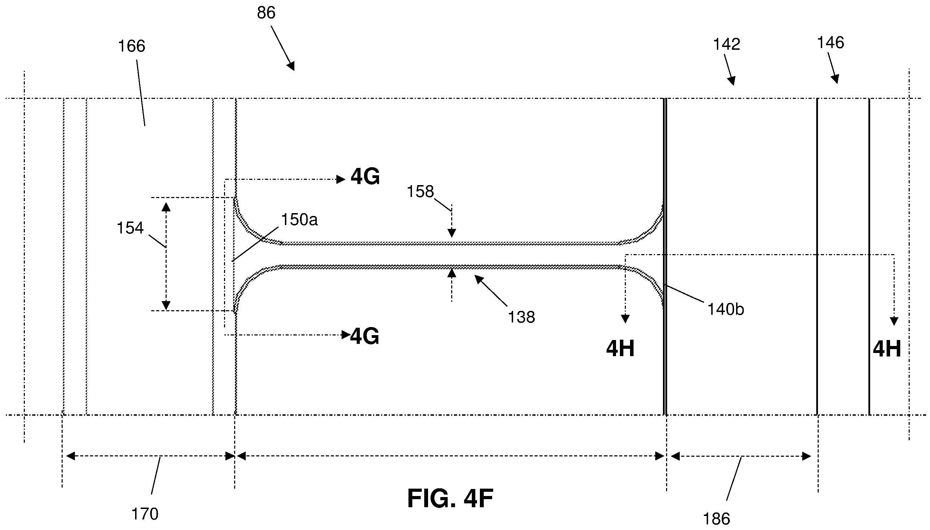

The droplet-generating region(s) can be configured to form droplets in any suitable manner. For example, referring additionally to FIGs. 4F-4H, for each of the flow path(s) a minimum cross-sectional area of the flow path can increase along the flow path in at least one of the droplet-generating region(s). To illustrate, in the droplet-generating region, the flow path can include a constricting section (e.g., 138), a constant section (e.g., 142), and/or an expanding section (e.g., 146).

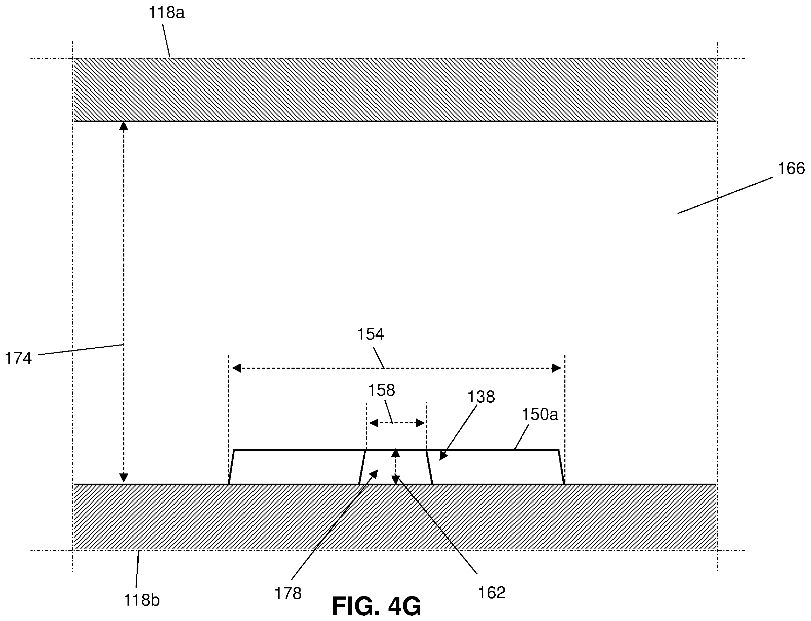

The constricting section can be configured to facilitate droplet generation. As shown, for example, the constricting section can extend between an inlet and an outlet (e.g., 150a and 150b), the inlet being connected to a channel (e.g., 166) such that liquid can enter the constricting section from the channel (FIGs. 4F and 4G). The channel can have a maximum transverse dimension (e.g., 170), taken perpendicularly to the centerline of the portion of the channel, and/or a maximum depth (e.g., 174), taken perpendicularly to the centerline and the transverse dimension thereof, that are larger than a maximum transverse dimension (e.g., 154) and maximum depth (e.g., 162), respectively, of the constricting section. For example, at least one of the channel's maximum transverse dimension and maximum depth can be greater than or equal to any one of, or between any two of, 10, 25, 50, 75, 100, 125, 150, 175, or 200 µm (e.g., between 75 and 170 µm), while the constricting section's maximum transverse dimension can be less than or equal to any one of, or between any two of, 200, 175, 150, 125, 100, 75, or 50 µm and maximum depth can be less than or equal to any one of, or between any two of, 20, 15, 10, or 5 µm. And, the constricting section can define a constriction between the inlet and outlet at which a cross-sectional area (e.g., 178) of the constricting section, taken perpendicularly to a centerline thereof, can be smaller (e.g., at least 10% smaller) than at the inlet and/or outlet. A minimum transverse dimension (e.g., 158) of the constricting section (e.g., at the constriction) can be less than or equal to any one of, or between any two of, 40, 35, 30, 25, 20, or 15 µm, and a length (e.g., 160) of the constricting section between its inlet and outlet can be greater than or equal to any one of, or between any two of, 250, 300, 350, 400, 450, 500, 550, 600, 650, 700, or 750 µm (e.g., between 450 and 750 µm), which can ensure the constricting section remains primed during droplet pinch-off.

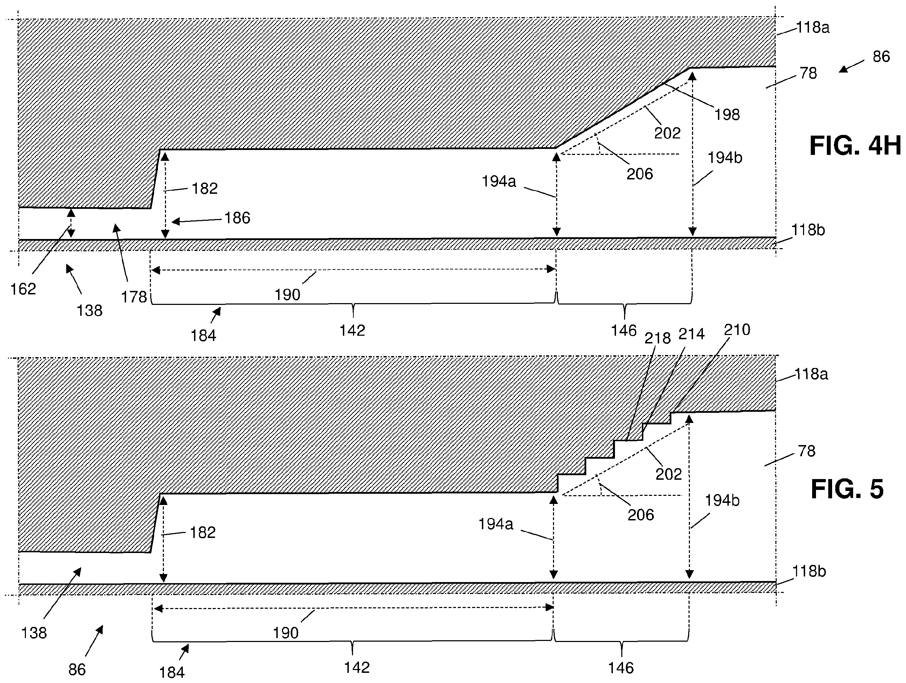

Droplet formation can be achieved by expanding the liquid following constriction thereof. Along the flow path, liquid from the constricting section can enter an expansion region (e.g., 184) in which a minimum cross-sectional area (e.g., 186) of the flow path is larger than the minimum cross-sectional area of the flow path in the constricting section (FIG. 4H). For example, the flow path's minimum cross-sectional area in the expansion region can be at least 10%, 50%, 100%, 200%, 300%, 400%, 500%, or 1,000% larger than its minimum cross-sectional area in the constricting section. Such an expansion may include variations in the depth of the flow path. A depth (e.g., 182, 194a, and/or 194b) of the flow path in the expansion region can be at least 10%, 50%, 100%, 150%, 200%, 250%, or 400% larger than the maximum depth of the constricting section, such as, for example, greater than or equal to or between any two of 5, 15, 30, 45, 60, 75, 90, 105, or 120 µm (e.g., between 35 and 45 µm or between 65 and 85 µm). Liquid flowing along the flow path from the constricting section into the expansion region can thereby expand and form droplets.

These depth variations can occur in a constant section and/or an expanding section of the flow path, where liquid flowing from one of the inlet port(s) to the test volume is permitted to exit the constricting section into the constant and/or expanding sections. In the embodiment shown in FIG. 4H, expansion of the liquid can be achieved with both a constant section and an expanding section, the geometry of which can promote the formation of droplets of substantially the same size and facilitate a suitable droplet arrangement in the test volume. The constant section and expanding section can be arranged such that fluid flowing from one of the inlet port(s) to the test volume is permitted to flow from constricting section, through the constant section, and to the expanding section. The constant section can have a depth (e.g., 182) that can be equal to the minimum depth of the expansion region and is larger (e.g., at least 10% or at least 50% larger) than the maximum depth of the constricting section, such as greater than or equal to any one of or between any two of 5, 20, 35, 50, 65, or 80 µm (e.g., between 35 and 45 µm). The depth of the constant section can be substantially the same along at least 90% of a length (e.g., 190) thereof between the constricting and expanding sections. The constant section can have any suitable length to permit complete droplet formation (including droplet pinch off), such as, for example, a length that is greater than or equal to any one of, or between any two of, 15, 25, 50, 100, 200, 300, 400, or 500 µm (e.g., between 150 and 200 µm).

The expanding section can expand such that, moving along the flow path toward the test volume, the depth of the expanding section increases from a first depth (e.g., 194a) to a second depth (e.g., 194b). The first and second depths can be, for example, the minimum and maximum depths of the expansion region, respectively. To illustrate, the expanding section can define a ramp (e.g., 198) having a slope (e.g., 202) that is angularly disposed relative to the constricting section by an angle (e.g., 206) such that the depth of the expanding section increases moving away from the constant section. That angle can be greater than or equal to any one of, or between any two of, 5°,10°, 20°, 30°, 40°, 50°, 60°, 70°, or 80° (e.g., between 20° and 40°), as measured relative to a direction parallel to the centerline of the constricting section. The ramp can extend from the constant section (e.g., such that the first depth is substantially the same as the constant section's depth) to a point at which the expansion region reaches its maximum depth, which can be greater than or equal to any one of, or between any two of, 15, 30, 45, 60, 75, 90, 105, or 120 µm (e.g., between 65 and 85 µm). As shown, the ramp is defined by a (e.g., single) planar surface. Referring to FIG. 5, however, in other embodiments the ramp can be defined by a plurality of steps (e.g., 210) (e.g., if the chip is made with a lithographically-produced mold, which can be cost-effective), each having an appropriate rise (e.g., 214) and run (e.g., 218) such that the ramp has the any of the above-described slopes.

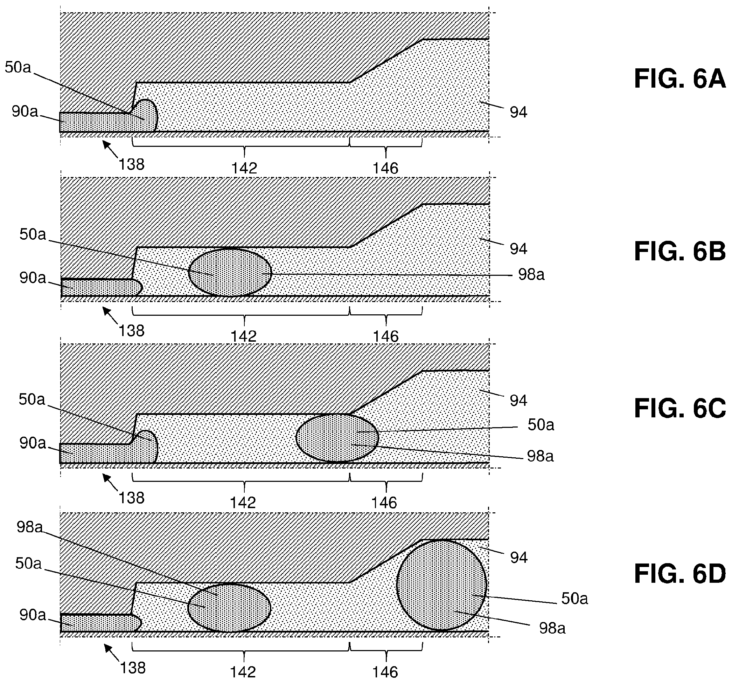

Referring additionally to FIGs. 6A-6D-which illustrate droplet formation using the constricting, constant, and expanding sections as described with respect to FIG. 4H-as sized, the constant section can compress the droplets to prevent full expansion thereof (FIGs. 6A and 6B). The constant section can thereby prevent the droplets from stacking on one another such that the droplets can be arranged in a two-dimensional array in the test volume. Such an array can facilitate accurate analysis of the droplets. Compressed droplets flowing from the constant section to the expanding section can travel and decompress along the ramp (FIGs. 6C and 6D). The decompression can lower the surface energy of the droplet such that the droplet is propelled along the ramp and out of the expanding section. At least by propelling droplets out of the expanding section, the ramp can mitigate droplet accumulation at the interface between the outlet of the constricting section and the constant section such that the droplets do not obstruct subsequent droplet formation. Because such obstruction can cause inconsistencies in droplet size, the expanding section-by mitigating blockage-can facilitate formation of consistently-sized droplets, e.g., droplets that each have a diameter within 3-6% of the diameter of each other of the droplets.

The droplet-generating region(s) can have other configurations to form droplets. For example, expansion of the liquid can be achieved with a constant section alone, an expanding section alone, or an expanding section upstream of a constant section. And while droplet generation can be achieved through expansion, in other embodiments the droplet-generating region(s) can be configured to form droplets in any suitable manner, such as via a T-junction (e.g., at which two channels-the first portion of the sample flowing through one and the non-aqueous liquid flowing through the other-connect such that the non-aqueous liquid shears the sample-containing liquid to form droplets), flow focusing, co-flow, and/or the like. In some of such alternative embodiments, the microfluidic network can include multiple inlet ports and the first portion of the sample and the non-aqueous liquid can be disposed in different inlet ports (e.g., such that they can meet at a junction for droplet generation). Other droplet generating techniques that do not use a microfluidic chip can be used as well.

Referring to FIGs. 7A-7C, phenotypic analysis of the target species can be performed using the second portion of the sample. The second portion of the sample can be a portion of the sample that was disposed in one of the inlet port(s) of the first chip and not used to generate the first droplets or a portion of the sample that was not introduced into the first chip. The second portion of the sample can be divided into one or more, optionally two or more, aliquots (e.g., 230). When the second portion of the sample is divided into multiple aliquots, one or more of the aliquots can be exposed to different test reagents with at least one of the aliquots not exposed to a test reagent to act as a control (e.g., to determine which of the test reagents provides the desired phenotypic response).

The analysis of the second portion of the sample can be performed using droplet microfluidics-for each of the aliquot(s), some methods include a step 22 of generating, with the second device, a plurality of second droplets (e.g., 98b) from a second liquid (e.g., 90b) that comprises the aliquot (which can be an aqueous liquid). This droplet generation can be performed in substantially the same manner as described above with respect to the first droplets. For example, the second portion of the sample can be introduced into an injection port (e.g., 222) of the second device (FIG. 7A) and partitioned into the aliquot(s), which can be communicated through one or more channels (e.g., 226) to a respective one of one or more microfluidic networks defined by one or more second chips (e.g., 66b) (FIG. 7B). Each of the second chip(s) can be substantially the same as the first chip (e.g., the microfluidic network(s) defined by the second chip(s) can be any of those described above) and, optionally, can be pre-loaded with a non-aqueous liquid such that that the second liquid includes the aliquot and the non-aqueous liquid. For each of the aliquot(s), the second liquid can be disposed in at least one of the inlet port(s) and directed along the flow path(s), through at least one droplet-generating region, and to the test volume for analysis (FIG. 7C). As a result, each of one or more microorganisms of the aliquot can be encapsulated within one of the second droplets.

Some methods include a step 26 of, for at least one of the aliquot(s), introducing a test reagent into at least some of the second droplets, optionally where for at least one of the aliquot(s) a test reagent is not introduced into the second droplets (e.g., to act as a control). This can be performed by introducing the test reagent into the aliquot (e.g., by pre-loading the microfluidic network with the test reagent or adding the test reagent to the aliquot before it reaches the microfluidic network) such that at least some of the second droplets, when generated, include the test reagent. Alternatively, droplets can be formed from the test reagent and merged with the second droplets.

The test reagent can be selected based on the phenotypic response under investigation. For example, when determining an appropriate treatment for a patient, the test reagent can comprise a drug such as an antibiotic (e.g., an antibacterial or an antifungal). When the test reagent comprises an antibiotic, the phenotypic response for analysis can include the susceptibility of the target species to the antibiotic. To illustrate, when multiple aliquots are used each of the aliquots can be exposed to a different antibiotic to determine which of the antibiotics is most effective at killing or inhibiting the growth of the target species. A test reagent need not be introduced into the second droplets formed from at least one of the aliquots-the aliquot(s) whose droplets do not include a test reagent can function as a control for the phenotypic analysis described below.

Some methods include a step 30 of capturing, with one or more sensors (e.g., 106), a second set of data indicative of a phenotypic response of the encapsulated microorganism(s) of the second portion of the sample to each of the test reagent(s). The second set of data can be captured in substantially the same manner as the first set of data. For example, the second liquid includes a viability indicator (e.g., resazurin) such that the encapsulating second droplets (e.g., 102b) exhibit a characteristic signature that varies over time (e.g., fluorescence over a second time period) based on the species of microorganism(s) encapsulated therein. The test reagent can affect the signature. To illustrate, when the test reagent comprises an antibiotic, the antibiotic may kill or inhibit the growth of the encapsulated microorganism(s) such that droplet conditions-and thus the characteristics of the viability indicator-differ from those that would exist without the test reagent. As an example, when the viability indicator comprises resazurin, a droplet including an antibiotic that kills encapsulated microorganism(s) may have a fluorescence similar to that of a droplet that does not encapsulate any microorganisms.

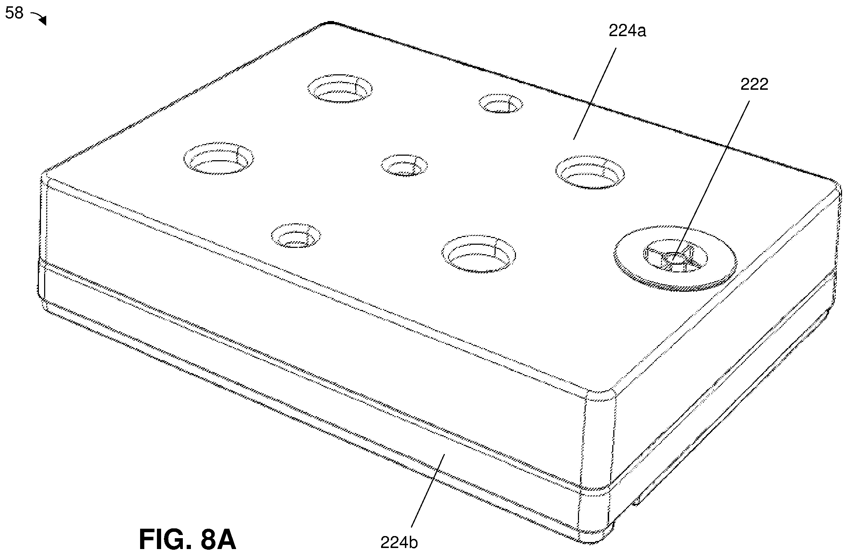

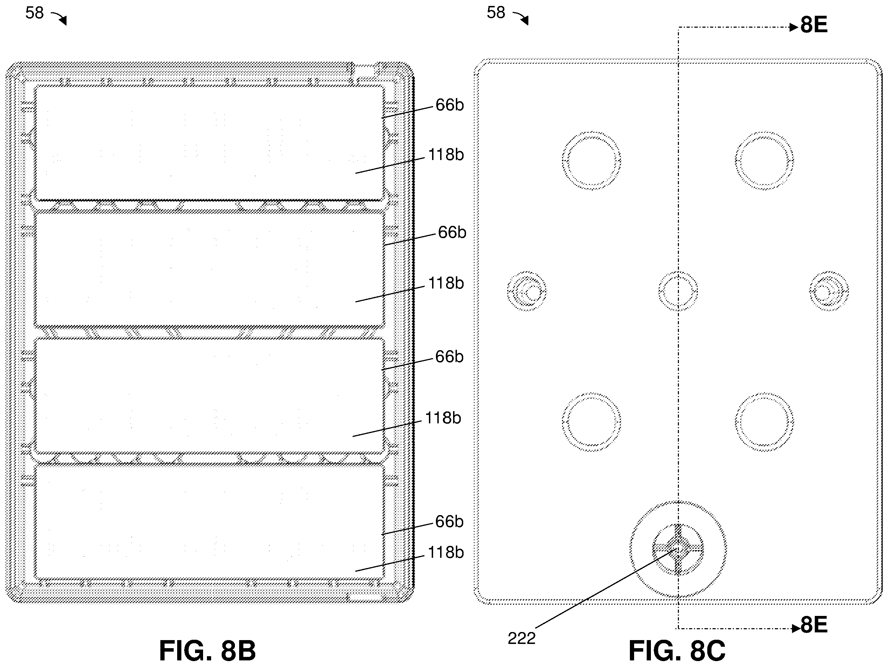

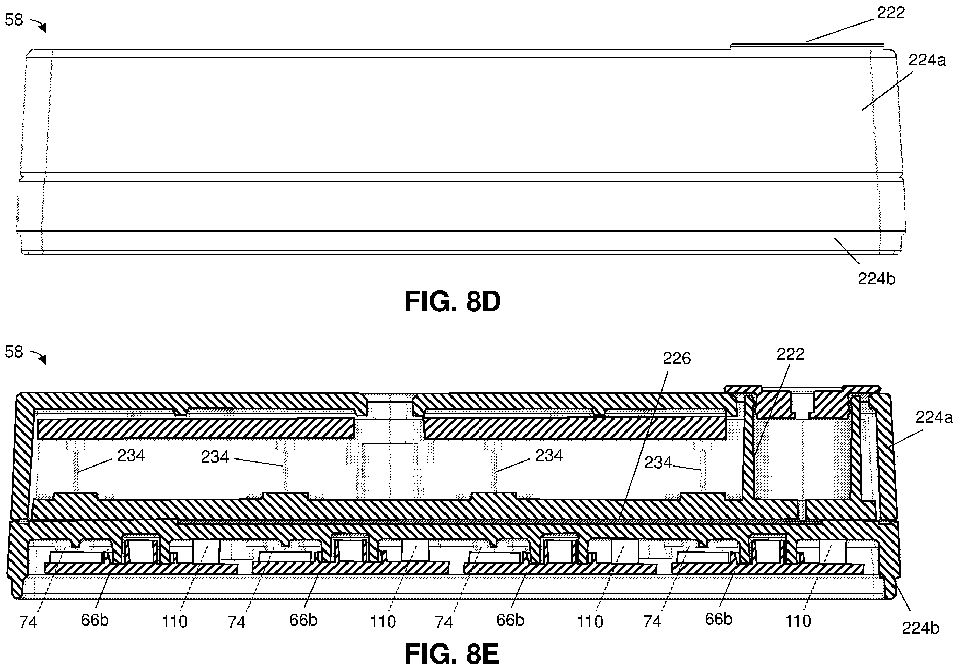

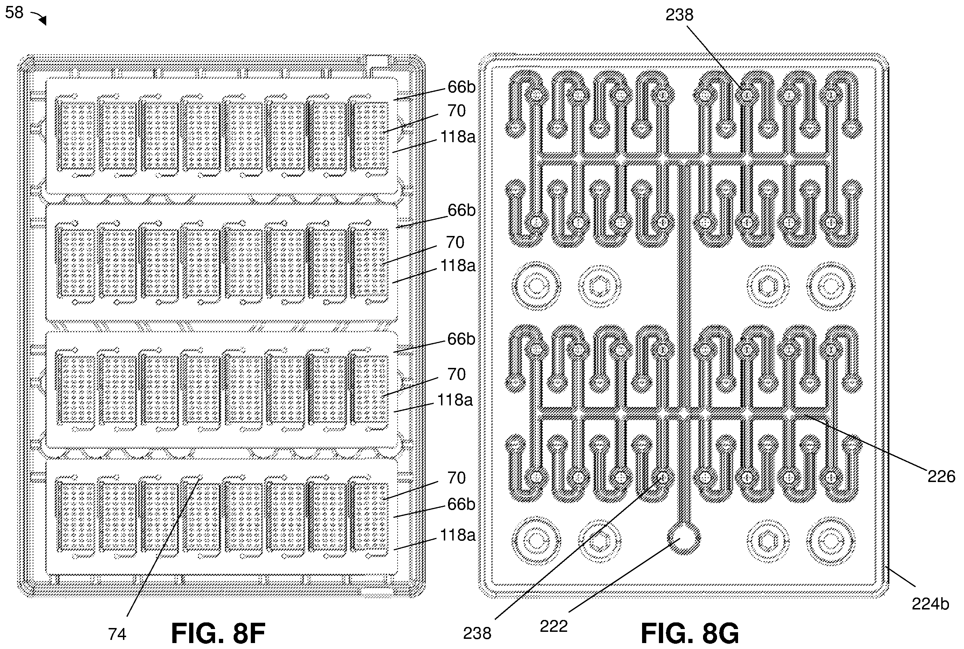

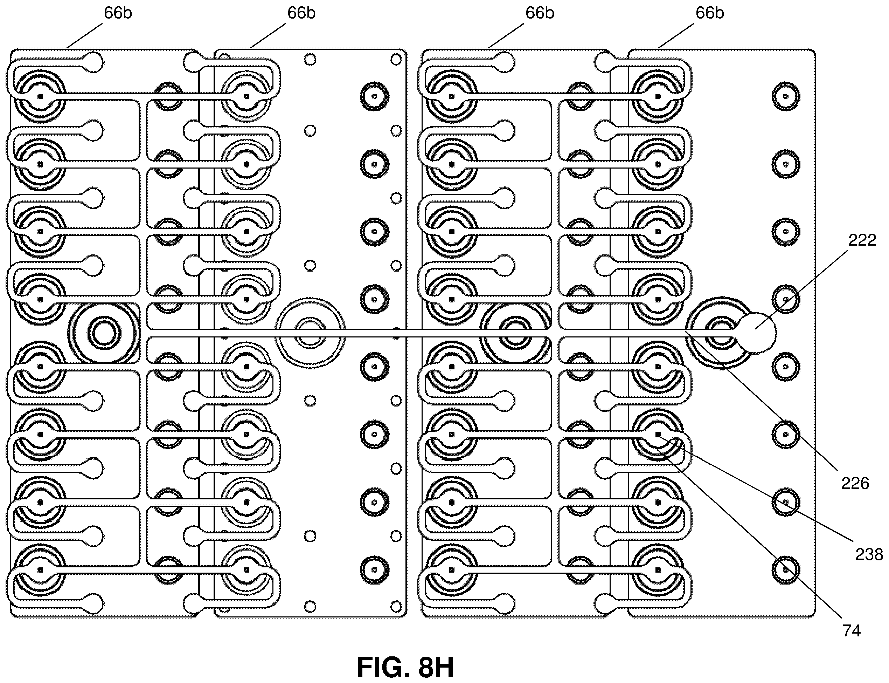

Referring to FIGs. 8A-8H, shown is an illustrative second device that can be used to partition the second portion of the sample into the aliquot(s), generate the second droplets from each of the aliquot(s), and capture the second set of data. The second device can include upper and lower pieces (e.g., 224a and 224b) and multiple microfluidic chips. As shown, the second device comprises four chips, each defining eight microfluidic networks such that the chips collectively define thirty two microfluidic networks. The device accordingly can be used to assess the effect of up to thirty two different test reagents (or thirty one with a control) on the encapsulated microorganism(s).

Each of the microfluidic networks of the chips can be pre-loaded with the non-aqueous liquid and/or a test reagent. To prevent loss thereof, the inlet port of each of the networks can be sealed. The second device can include a piercer (e.g., 234) for each of the inlet ports-each of the piercers can be configured to break the seal of a respective one of the inlet ports such that one of the aliquots can be introduced therein (FIG. 8E). The channels of the second device can be defined by the lower piece of the second device and can extend between the injection port and a plurality of outlets (e.g., 238), each of which permits an aliquot to be transferred to one of the microfluidic networks. For example, each of the outlets of the second device can be aligned with a respective one of the inlet ports of the microfluidic networks such that that liquid can flow from the injection port, through at least one of the channels to one of the outlet ports, and into one of the microfluidic networks (FIGs. 8F-8H).

Some methods include a step 34 of determining a phenotypic response of the target species to each of the test reagent(s). Because the phenotypic analysis can be performed after the initial screen-which may take one or more hours-and the microorganism(s) can replicate during that time, the concentration of microorganism(s) in the second portion of the sample may be different from that in the original sample. This can pose challenges for samples taken from non-sterile sites, which may include multiple species of microorganisms that have different replication rates. For example, a commensal (e.g., non-target) microorganism having a relatively fast replication rate may appear to be pathogenic (e.g., a target species) in the second portion of the sample due at least in part to that replication rate (e.g., which can yield higher concentrations of the commensal microorganism). The second set of data, alone, may thus be insufficient to ascertain which of the measurements are relevant (e.g., the measurements that illustrate the phenotypic response of the target, rather than non-target, species).

To address these challenges, the phenotypic response of the target species to the test reagent(s) can be determined at least by referencing the second set of data to the first set of data. Because the first set of data may reflect the original microorganism concentrations, referencing that data can facilitate interpretation of the second set of data such that the effect of the test reagent(s) on the target species can be ascertained and distinguished from their effect on any non-target species. For example, the first set of data can be referenced to determine which of the species is a target species and thus the characteristic signature (e.g., fluorescent signature) that is relevant for the analysis. Data indicating that for second droplets into which a test reagent was introduced there is a deviation from the relevant characteristic signature-regardless of whether there is a deviation in the characteristic signature of encapsulating droplet(s) that include non-target species-can evidence that the test reagent affects the target species.

To determine whether there is a deviation, the second set of data can include control data captured from second droplets formed from an aliquot where a test reagent was omitted, as described above. That control data can be indicative of the quantity of the encapsulated microorganism(s) that exist when not exposed to the test reagent. The data captured from the second droplets formed from the other aliquot(s)-into which a test reagent was introduced-can be referenced to the control data along with the first set of data to determine the effect of the test reagent(s) on the target species. For example, when data obtained from the analysis of the non-control aliquot(s) shows that for at least one of those aliquot(s) there is a deviation in the characteristic signature of the target species relative to the control (e.g., if there are fewer droplets exhibiting the relevant characteristic signature), it can be determined that the test reagent affects the target species. As an illustration, when the test reagent comprises an antibiotic and the relevant characteristic signature is not measured or fewer droplets exhibit the relevant characteristic signature compared to the control, it can be determined that the target species is susceptible to the antibiotic (e.g., because the characteristic signature of the target species, if alive and allowed to propagate, would have been detected in greater quantities) even if the antibiotic does not kill or inhibit the growth of non-target species. This cross-referencing is achievable at least in part because the first and second portions of the sample can be analyzed using droplet microfluidics, where each of the encapsulating first and second droplets can encapsulate a single species to yield the unique, characteristic signatures that permit differentiation.

This method of phenotypic analysis can be more accurate and efficient than conventional techniques. For example, because the microfluidic analysis is phenotypic (e.g., it directly measures the response of the target species to the test reagent), it can more accurately assess the effect of the test reagent (e.g., its effectiveness as an antibiotic) than genotypic techniques such as NAATs, which indirectly make these assessments based on genetic information. For example, genotypic techniques may not be able to account for mutations (e.g., evolution in resistance mechanisms). Additionally, by using droplet microfluidics, the phenotypic analysis can be faster and more efficient than conventional phenotypic tests such as microdilution and disk diffusion. Those tests may require additional culturing of the sample and isolation of the target species (e.g., by streaking the sample across a plate), which can be both time- and work-intensive. As described above, due to the low volume of each of the encapsulating droplets, the concentration of microorganism(s) therein can be relatively high such that additional culturing is unnecessary. And because droplet formation isolates the different species of microorganisms by encapsulating them such that the species can be differentiated, isolation of the target species before the phenotypic analysis (e.g., before an AST) may be unnecessary as well such that the analysis can be performed in significantly less time.

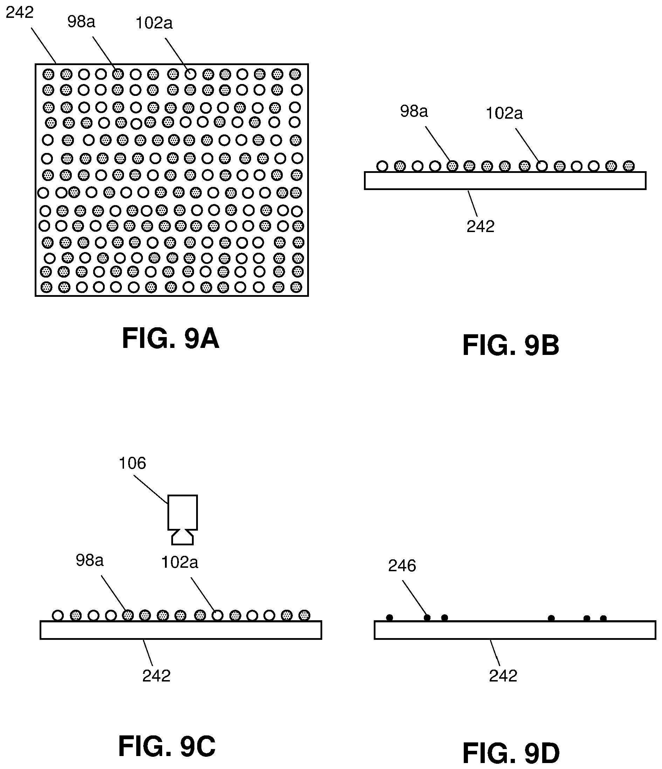

Referring additionally to FIGs. 9A-9G, after the initial screen the sample can be analyzed further using mass spectrometry to provide higher resolution classification of the target species. This analysis can be performed using some of the first droplets. Some methods include a step 38 of removing at least some of the first droplets from the first device (e.g., from the outlet port(s) of the first chip), and, optionally, disposing the removed first droplets on a plate (e.g., 242) (FIGs. 9A and 9B). The removed first droplets can include at least some of the encapsulated microorganisms of the first portion of the sample and can be disposed on the plate such that the droplets form an array for analysis thereof.

The location, on the plate, of one(s) of the removed first droplets that include encapsulated microorganism(s) can be determined. For example, a sensor (e.g., 106), such as an imaging sensor, can capture data-such as fluorescence measurements-indicative of the location of droplets that encapsulate the target species (FIG. 9C). This location information can be used to determine where to initially scan with the mass spectrometer to accelerate the analysis.

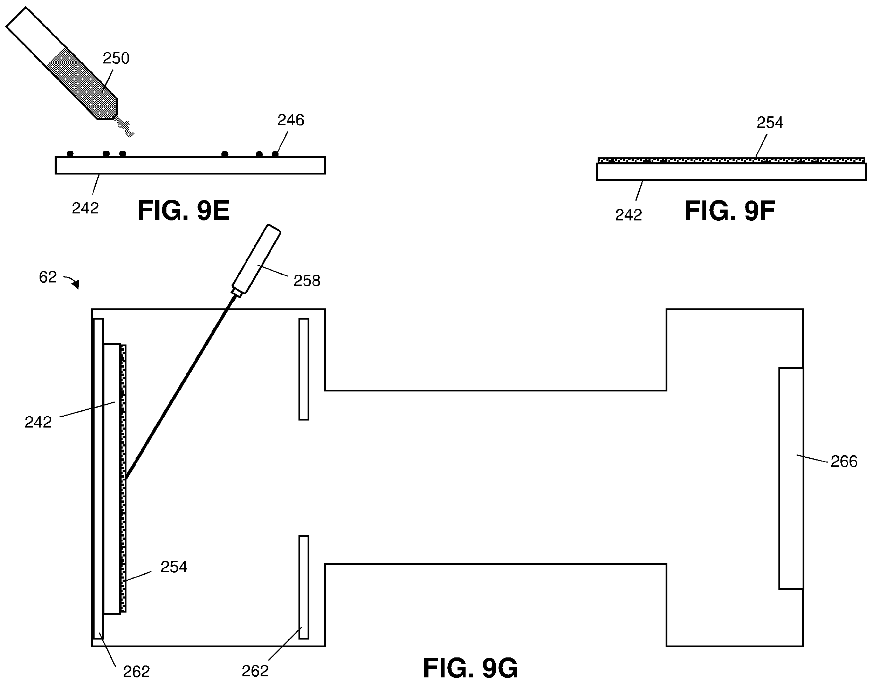

The removed first droplets can be dried on the plate such that substantially all of the liquid of the removed first droplet evaporates (e.g., by waiting for such evaporation to occur) (FIG. 9D). Due to the relatively high concentration of microorganism(s) in each of the removed first droplets, after the droplets are dried concentrated spots of microorganism(s) (e.g., 246) can remain on the plate where the encapsulating droplet(s) were disposed. One or more lysing reagents (e.g., 250) can be added to the plate to lyse the microorganism(s) disposed thereon (FIG. 9E), however in certain instances a lysis step may not be required. The spectrometry analysis can be performed using matrix assisted laser desorption/ionization time of flight (MALDI-TOF) mass spectrometry in which the microorganism(s) are ionized. In preparation for that analysis, a matrix material (e.g., 254), such as sinapinic acid, CHCA, or DHB, can be added to the plate (e.g., such that it is mixed with the microorganism(s)) (FIG. 9F).

Some methods include a step 42 of capturing spectrometry data, with the mass spectrometer, indicative of the identity of the encapsulated microorganism(s) of the removed first droplets (e.g., the identity of the target species). The spectrometry data can be captured by analyzing the ionized microorganism(s) while they are disposed on the plate (FIG. 9G). As shown, the mass spectrometer is a MALDI-TOF mass spectrometer including a laser (e.g., 258), one or more electric field generators (e.g., 262), and a detector (e.g., 266). The laser can be directed onto the plate such the microorganism(s) and matrix material are ionized and ejected from the plate. The ejected material can move to the detector under the influence of an electric field generated by the electric field generator(s). The time of flight of ejected particles (e.g., as determined from data captured by the detector)-which may depend on the mass-to-charge ratio of the particles-can be used to generate the spectrometry data. The mass spectrometry can provide a higher resolution analysis of the identity of the target species.

## Claims

1. A method of analyzing a sample comprising one or more species of microorganisms, the method comprising:
generating, with a first microfluidic device, a plurality of first droplets from a first liquid that comprises a viability indicator and a first portion of the sample such that each of one or more microorganisms of the first portion of the sample is encapsulated within one of the first droplets;
capturing, with one or more sensors, a first set of data indicative of the identity and quantity of the encapsulated microorganism(s) of the first portion of the sample;
identifying at least one of the one or more species of the sample as a target species based on the first set of data;
for each of one or more aliquots of a second portion of the sample, generating, with a second microfluidic device, a plurality of second droplets from a second liquid that comprises a viability indicator and the aliquot such that each of one or more microorganisms of the aliquot is encapsulated within one of the second droplets;
for at least one of the aliquot(s), introducing a test reagent into at least some of the second droplets;
capturing, with one or more sensors, a second set of data indicative of a phenotypic response of the encapsulated microorganisms(s) of the second portion of the sample to each of the test reagent(s); and
determining a phenotypic response of the target species to each of the test reagent(s) at least by referencing the second set of data to the first set of data,
wherein if the analysis with the first microfluidic device does not identify the target species in the sample, then the sample is not analyzed with the second device.

2. The method of claim 1, wherein the first liquid comprises a broth.

3. The method of claim 1 or 2, wherein the one or more aliquots comprise two or more aliquots.

4. The method of any one of claims 1-3, wherein the viability indicator of the first liquid and the viability indicator of the second liquid each comprise resazurin.

5. The method of any of claims 1-4, wherein at least one of the first and second liquids comprises a non-aqueous liquid.

6. The method of claim 5, wherein the non-aqueous liquid has a specific gravity that is greater than or equal to 1.2.

7. The method of any of claims 1-6, wherein identifying at least one of the one or more species as a target species comprises, for each of the one or more species:
calculating a concentration of the species in the sample based on the first set of data; and
if the concentration is greater than or equal to a threshold concentration, identifying the species as a target species.

8. The method of any of claims 1-7, wherein the first set of data comprises measurements of the fluorescence of at least some of the first droplets over a first test period.

9. The method of any of claims 1-8, wherein the second set of data comprises measurements of the fluorescence of at least some of the second droplets over a second test period.

10. The method of any of claims 1-9, wherein for at least one of the aliquot(s) introducing the test reagent into the second droplets comprises introducing the test reagent into the aliquot.

11. The method of any of claims 1-10, wherein:
each of the test reagent(s) comprises an antibiotic; and
the phenotypic response of the target species to each of the test reagent(s) comprises susceptibility of the target species to the antibiotic.

12. The method of any of claims **1-11,** wherein:
the first microfluidic device comprises a first chip defining a microfluidic network that includes:
one or more inlet ports;
a test volume; and
one or more flow paths extending between the inlet port(s) and the test volume; and generating the first droplets is performed in the microfluidic network of the first chip at
least by:
disposing the first liquid within a first one of the inlet port(s); and
directing the first liquid along the flow path(s) such that, for each of the flow path(s), at least a portion of the first liquid flows from the first inlet port, through at least one droplet-generating region in which a minimum cross-sectional area of the flow path increases along the flow path, and to the test volume; and
capturing the first set of data comprises analyzing the first droplets that are disposed in the test volume.

13. The method of any of claims 1-12, wherein:
the second microfluidic device comprises a second chip comprising one or more microfluidic networks, each including:
one or more inlet ports;
a test volume; and
one or more flow paths extending between the inlet port(s) and the test volume; and
for each of the aliquot(s) generating the second droplets is performed in a respective one of the microfluidic network(s) of the second chip at least by:
disposing the second liquid within a first one of the inlet port(s) of the microfluidic network; and
directing the second liquid along the flow path(s) such that, for each of the flow path(s), at least a portion of the second liquid flows from the first inlet port, through at least one droplet-generating region in which a minimum cross-sectional area of the flow path increases along the flow path, and to the test volume; and
capturing the second set of data comprises analyzing the second droplets that are disposed in each of the test volume(s).

14. The method of claim 12 or 13, wherein for the first microfluidic chip:
the microfluidic network comprises:
one or more outlet ports; and
one or more outlet channels in fluid communication between the test volume and the outlet port(s);
generating the first droplets is performed such that at least some of the first droplets flow from the test volume, through the outlet channel(s), and into the outlet port(s); and
the method comprises removing at least some of the first droplets from the outlet port(s).

15. The method of any of claims 1-14, wherein the sample comprises two or more species of microorganisms.

## Patentansprüche

1. Verfahren zum Analysieren einer Probe, die eine oder mehrere Spezies von Mikroorganismen umfasst, bei dem:
mit einer ersten mikrofluidischen Vorrichtung eine Vielzahl von ersten Tröpfchen aus einer ersten Flüssigkeit generiert wird, die einen Lebensfähigkeitsindikator und einen ersten Anteil der Probe umfasst, so dass jeder des einen oder der mehreren Mikroorganismen des ersten Anteils der Probe in einem der ersten Tröpfchen verkapselt ist,
mit einem oder mehreren Sensoren ein erster Satz von Daten erfasst wird, der die Identität und Quantität des verkapselten Mikroorganismus bzw. der verkapselten Mikroorganismen des ersten Anteils der Probe angibt,
mindestens eine der einen oder mehreren Spezies der Probe basierend auf dem ersten Satz von Daten als Zielspezies identifiziert wird,
für jedes von einem oder mehreren Aliquoten eines zweiten Anteils der Probe mit einer zweiten mikrofluidischen Vorrichtung eine Vielzahl von zweiten Tröpfchen aus einer zweiten Flüssigkeit generiert wird, die einen Lebensfähigkeitsindikator und das Aliquot umfasst, so dass jeder von dem einen oder den mehreren Mikroorganismen des Aliquots in einem von den zweiten Tröpfchen verkapselt ist,
für mindestens einen der Aliquote(n) ein Testreagenz in mindestens einige der zweiten Tröpfchen eingebracht wird,
mit einem oder mehreren Sensoren ein zweiter Satz von Daten erfasst wird, der eine phänotypische Reaktion des verkapselten Mikroorganismus bzw. der verkapselten Mikroorganismen des zweiten Anteils der Probe auf das Testreagenzes bzw. jedes der Testreagenzien angibt, und
eine phänotypische Reaktion der Zielspezies auf das Testreagenzes bzw. jedes der Testreagenzien mindestens bestimmt wird, indem der zweite Satz der Daten auf den ersten Satz der Daten referenziert wird,
wobei, falls die Analyse mit der ersten mikrofluidischen Vorrichtung die Zielspezies in der Probe nicht identifiziert, dann die Probe nicht mit der zweiten Vorrichtung analysiert wird.

2. Verfahren nach Anspruch 1, bei dem die erste Flüssigkeit eine Bouillon umfasst.

3. Verfahren nach Anspruch 1 oder 2, bei dem das eine oder die mehreren Aliquoten zwei oder mehr Aliquoten umfassen.

4. Verfahren nach einem der Ansprüche 1 bis 3, bei dem der Lebensfähigkeitsindikator der ersten Flüssigkeit und der Lebensfähigkeitsindikator der zweiten Flüssigkeit jeweils Resazurin umfasst.

5. Verfahren nach einem der Ansprüche 1 bis 4, bei dem mindestens eine der ersten und der zweiten Flüssigkeit eine nicht-wässrige Flüssigkeit umfasst.

6. Verfahren nach Anspruch 5, bei dem die nicht-wässrige Flüssigkeit ein spezifisches Gewicht aufweist, das größer als oder gleich 1,2 ist.

7. Verfahren nach einem der Ansprüche 1 bis 6, bei dem Identifizieren von mindestens einem der einen oder mehreren Spezies als Zielspezies für jede der einen oder mehreren Spezies umfasst, dass:
eine Konzentration der Spezies in der Probe basierend auf dem ersten Satz von Daten berechnet wird, und, falls die Konzentration größer als oder gleich einer Schwellenkonzentration ist, die Spezies als Zielspezies identifiziert wird.

8. Verfahren nach einem der Ansprüche 1 bis 7, bei dem der erste Satz von Daten Messungen der Fluoreszenz von mindestens einigen der ersten Tröpfchen über eine erste Testperiode umfasst.

9. Verfahren nach einem der Ansprüche 1 bis 8, bei dem der zweite Satz von Daten Messungen der Fluoreszenz von mindestens einigen der zweiten Tröpfchen über eine zweite Testperiode umfasst.

10. Verfahren nach einem der Ansprüche 1 bis 9, bei dem für mindestens eine der Aliquote(n) Einbringen des Testreagenzes in die zweiten Tröpfchen Einbringen des Testreagenzes in die Aliquote umfasst.

11. Verfahren nach einem der Ansprüche 1 bis 10, bei dem:
jedes von dem Testreagenz bzw. den Testreagenzien ein Antibiotikum umfasst, und
die phänotypische Reaktion der Zielspezies auf jedes des Testreagenzes bzw. der Testreagenzien Empfindlichkeit der Zielspezies gegenüber dem Antibiotikum umfasst.

12. Verfahren nach einem der Ansprüche 1 bis 11, bei dem:
die erste mikrofluidische Vorrichtung einen ersten Chip umfasst, der ein mikrofluidisches Netzwerk definiert, welches folgendes einschließt:
eine oder mehrere Einlassöffnungen,
ein Testvolumen und
einen oder mehrere Fließpfade, der bzw. die sich zwischen der Einlassöffnung bzw. den Einlassöffnungen und dem Testvolumen erstreckt bzw. erstrecken, und
Generieren der ersten Tröpfchen in dem mikrofluidischen Netzwerk des ersten Chips durchgeführt wird, indem mindestens:
die erste Flüssigkeit in einer ersten der Einlassöffnung(en) angeordnet wird, und
die erste Flüssigkeit den Fließpfad bzw. die Fließpfade entlang gelenkt wird, so dass bei jedem des Fließpfads bzw. der Fließpfade mindestens ein Anteil der ersten Flüssigkeit von der ersten Einlassöffnung durch mindestens eine Tröpfchengenerierungsregion, bei der eine Mindestquerschnittfläche des Fließpfads entlang des Fließpfads zunimmt, und zu dem Testvolumen fließt, und
Erfassen des ersten Satzes von Daten Analysieren der ersten Tröpfchen umfasst, die in dem Testvolumen angeordnet sind.

13. Verfahren nach einem der Ansprüche 1 bis 12, bei dem:
die zweite mikrofluidische Vorrichtung einen zweiten Chip umfasst, der ein oder mehrere mikrofluidische Netzwerke umfasst, die jeweils folgendes einschließen:
eine oder mehrere Einlassöffnungen,
ein Testvolumen und
einen oder mehrere Fließpfade, der bzw. die sich zwischen der Einlassöffnung bzw. den Einlassöffnungen und dem Testvolumen erstreckt bzw. erstrecken, und
für jedes der Aliquote(n) Generieren der zweiten Tröpfchen in einem jeweiligen des mikrofluidischen Netzwerks bzw. der mikrofluidischen Netzwerke des zweiten Chips durchgeführt wird, indem mindestens:
die zweite Flüssigkeit in einer ersten der Einlassöffnung(en) des mikrofluidischen Netzwerks angeordnet wird, und
die zweite Flüssigkeit entlang des Fließpfads bzw. der Fließpfade gelenkt wird, so dass für jeden der Fließpfad(e) mindestens ein Anteil der zweiten Flüssigkeit von der ersten Einlassöffnung durch mindestens eine Tröpfchengenerierungsregion, bei der eine Mindestquerschnittfläche des Fließpfads entlang des Fließpfads zunimmt, und zu dem Testvolumen fließt, und
Erfassen des zweiten Satzes von Daten Analysieren der zweiten Tröpfchen umfasst, die in jedem des Testvolumens bzw. der Testvolumina angeordnet sind.

14. Verfahren nach Anspruch 12 oder 13, bei dem für den ersten mikrofluidischen Chip:
das mikrofluidische Netzwerk folgendes umfasst:
eine oder mehrere Auslassöffnungen und
einen oder mehrere Auslasskanäle in Fließverbindung zwischen dem Testvolumen und der Auslassöffnung bzw. den Auslassöffnungen,
wobei Generieren der ersten Tröpfchen so durchgeführt wird, dass mindestens einige der ersten Tröpfchen aus dem Testvolumen durch den Auslasskanal bzw. die Auslasskanäle hindurch und in die Auslassöffnung(en) fließen, und
das Verfahren Entfernen von mindestens einigen der ersten Tröpfchen aus der Auslassöffnung bzw. den Auslassöffnungen umfasst.

15. Verfahren nach einem der Ansprüche 1 bis 14, bei dem die Probe zwei oder mehr Spezies von Mikroorganismen umfasst.

## Revendications

1. Procédé d'analyse d'un échantillon comportant une ou plusieurs espèces de micro-organismes, le procédé comportant :
la génération, avec un premier dispositif microfluidique, d'une pluralité de premières gouttelettes à partir d'un premier liquide qui comporte un indicateur de viabilité et une première partie de l'échantillon de sorte que chacun d'un ou plusieurs microorganismes de la première partie de l'échantillon soit encapsulé au sein de l'une des premières gouttelettes ;
la capture, avec un ou plusieurs capteurs, d'un premier ensemble de données indiquant l'identité et la quantité du ou des micro-organisme(s) encapsulé(s) de la première partie de l'échantillon ;
l'identification d'au moins une des une ou plusieurs espèces de l'échantillon comme espèce cible sur la base du premier ensemble de données ;
pour chacune d'une ou plusieurs aliquotes d'une deuxième partie de l'échantillon, la génération, avec un deuxième dispositif microfluidique, d'une pluralité de deuxièmes gouttelettes à partir d'un deuxième liquide qui comporte un indicateur de viabilité et l'aliquote de sorte que chacun d'un ou plusieurs microorganismes de l'aliquote soit encapsulé au sein de l'une des deuxièmes gouttelettes ;
pour au moins une de la ou des aliquote(s), l'introduction d'un réactif d'essai dans au moins certaines des deuxièmes gouttelettes ;
la capture, avec un ou plusieurs capteurs, d'un deuxième ensemble de données indiquant une réponse phénotypique du ou des micro-organisme(s) encapsulé(s) de la deuxième partie de l'échantillon à chacun du ou des réactif(s) d'essai ; et
la détermination d'une réponse phénotypique de l'espèce cible à chacun du ou des réactif(s) d'essai au moins par référencement du deuxième ensemble de données au premier ensemble de données,
dans lequel si l'analyse avec le premier dispositif microfluidique n'identifie pas l'espèce cible dans l'échantillon, alors l'échantillon n'est pas analysé avec le deuxième dispositif.

2. Procédé selon la revendication 1, dans lequel le premier liquide comporte un bouillon.

3. Procédé selon la revendication 1 ou 2, dans lequel les une ou plusieurs aliquotes comportent deux aliquotes ou plus.

4. Procédé selon l'une quelconque des revendications 1 à 3, dans lequel l'indicateur de viabilité du premier liquide et l'indicateur de viabilité du deuxième liquide comportent chacun de la résazurine.

5. Procédé selon l'une quelconque des revendications 1 à 4, dans lequel au moins l'un des premier et deuxième liquides comporte un liquide non aqueux.

6. Procédé selon la revendication 5, dans lequel le liquide non aqueux a une densité spécifique supérieure ou égale à 1,2.

7. Procédé selon l'une quelconque des revendications 1 à 6, dans lequel l'identification d'au moins une des une ou plusieurs espèces comme espèce cible comporte, pour chacune des une ou plusieurs espèces :
le calcul d'une concentration de l'espèce dans l'échantillon sur la base du premier ensemble de données ; et
si la concentration est supérieure ou égale à une concentration seuil, l'identification de l'espèce comme espèce cible.

8. Procédé selon l'une quelconque des revendications 1 à 7, dans lequel le premier ensemble de données comporte des mesures de la fluorescence d'au moins certaines des premières gouttelettes sur une première période d'essai.

9. Procédé selon l'une quelconque des revendications 1 à 8, dans lequel le deuxième ensemble de données comporte des mesures de la fluorescence d'au moins certaines des deuxièmes gouttelettes sur une deuxième période d'essai.

10. Procédé selon l'une quelconque des revendications 1 à 9, dans lequel pour au moins l'une de la ou des aliquote(s) l'introduction de réactif d'essai dans les deuxièmes gouttelettes comporte l'introduction du réactif d'essai dans l'aliquote.

11. Procédé selon l'une quelconque des revendications 1 à 10, dans lequel :
chacun du ou des réactif(s) d'essai comporte un antibiotique ; et
la réponse phénotypique de l'espèce cible à chacun du ou des réactif(s) d'essai comporte une susceptibilité de l'espèce cible à l'antibiotique.

12. Procédé selon l'une quelconque des revendications 1 à 11, dans lequel :
le premier dispositif microfluidique comporte une première puce définissant un réseau microfluidique qui inclut :
un ou plusieurs orifices d'entrée ;
un volume d'essai ; et
un ou plusieurs trajets d'écoulement s'étendant entre le ou les orifice(s) d'entrée et le volume d'essai ; et
la génération des premières gouttelettes est réalisée dans le réseau microfluidique de la première puce au moins par :
disposition du premier liquide au sein d'un premier du ou des orifice(s) d'entrée ; et
fait de diriger le premier liquide le long du ou des trajet(s) d'écoulement de sorte que, pour chacun du ou des trajet(s) d'écoulement, au moins une partie du premier liquide s'écoule du premier orifice d'entrée, à travers au moins une région génératrice de gouttelettes dans laquelle une zone de section transversale minimale du trajet d'écoulement augmente le long du trajet d'écoulement, et vers le volume d'essai ; et
la capture du premier ensemble de données comporte l'analyse des premières gouttelettes qui sont disposées dans le volume d'essai.

13. Procédé selon l'une quelconque des revendications 1 à 12, dans lequel :
le deuxième dispositif microfluidique comporte une deuxième puce comportant un ou plusieurs réseaux microfluidiques, chacun comportant :
un ou plusieurs orifices d'entrée ;
un volume d'essai ; et
un ou plusieurs trajets d'écoulement s'étendant entre le ou les orifice(s) d'entrée et le volume d'essai ; et
pour chacune de la ou des aliquote(s) la génération des deuxièmes gouttelettes est réalisée dans un réseau respectif du ou des réseau(x) microfluidique(s) de la deuxième puce au moins par :
disposition du deuxième liquide au sein d'un premier du ou des orifice(s) d'entrée du réseau microfluidique ; et
fait de diriger le deuxième liquide le long du ou des trajet(s) d'écoulement de sorte que, pour chacun du ou des trajet(s) d'écoulement, au moins une partie du deuxième liquide s'écoule du premier orifice d'entrée, à travers au moins une région génératrice de gouttelettes dans laquelle une zone de section transversale minimale du trajet d'écoulement augmente le long du trajet d'écoulement, et vers le volume d'essai ; et
la capture du deuxième ensemble de données comporte l'analyse des deuxièmes gouttelettes qui sont disposées dans chacun du ou des volume(s) d'essai.

14. Procédé selon la revendication 12 ou 13, dans lequel pour la première puce microfluidique :
le réseau microfluidique comporte :
un ou plusieurs orifices de sortie ; et
un ou plusieurs canaux de sortie en communication fluidique entre le volume d'essai et le ou les orifice(s) de sortie ;
la génération des premières gouttelettes est réalisée de sorte qu'au moins certaines des premières gouttelettes s'écoulent du volume d'essai, à travers le canal ou les canaux de sortie, et dans le ou les orifice(s) de sortie ; et
le procédé comporte l'élimination d'au moins certaines des premières gouttelettes du ou des orifice(s) de sortie.

15. Procédé selon l'une quelconque des revendications 1 à 14, dans lequel l'échantillon comporte deux espèces ou plus de micro-organismes.
